Europäisches Patentamt

European Patent Office

Office européen des brevets

(11) Numéro de publication: **0 409 709 A1**

## DEMANDE DE BREVET EUROPEEN

(21) Numéro de dépôt: 90402039.3

(22) Date de dépôt: 16.07.90

(51) Int. Cl.⁵: **C07C 209/36, C07C 211/52**

(30) Priorité: 20.07.89 FR 8909766

(43) Date de publication de la demande:
23.01.91 Bulletin 91/04

(84) Etats contractants désignés:
AT BE CH DE DK ES FR GB GR IT LI LU NL SE

(71) Demandeur: **RHONE-POULENC CHIMIE**
**25, quai Paul Doumer**
**F-92408 Courbevoie Cédex(FR)**

(72) Inventeur: **Grosselin, Jean-Michel**
**15, rue Terraille**
**F-69001 Lyon(FR)**
Inventeur: **Baillard, Rosemarie**
**5, rue Molière**
**F-69006 Lyon(FR)**
Inventeur: **Cordier, Georges**
**50, chemin des Hermières**
**F-69340 Francheville(FR)**
Inventeur: **Langlois, Bernard**
**91 rue Duguesclin**
**F-69006 Lyon(FR)**
Inventeur: **Gilbert, Laurent**
**72, rue Vauban**
**F-69006 Lyon(FR)**
Inventeur: **Forat, Gérard**
**276, rue Duguesclin**
**F-69003 Lyon(FR)**

(74) Mandataire: **Ricalens, François et al**
**RHONE-POULENC CHIMIE Service Brevets**
**Chimie 25, quai Paul Doumer**
**F-92408 Courbevoie(FR)**

(54) **Procédé d'hydrogénation de dérivés halogéno nitroaromatiques en présence d'un dérivé soufré.**

(57) La présente invention concerne un procédé d'hydrogénation de dérivés halogénonitroaromatiques dans lequel on met en présence ledit dérivé avec du nickel de Raney et de l'hydrogène en présence d'un dérivé soufré.

EP 0 409 709 A1

# PROCEDE D'HYDROGENATION DE DERIVES HALOGENO NITROAROMATIQUES EN PRESENCE D'UN DERIVE SOUFRE

La présente invention concerne un procédé d'hydrogénation de composés aromatiques à la fois nitrés et halogénés . Elle concerne plus particulièrement la préparation d'amines halogénées.

Lors de la mise en oeuvre d'un procédé d'hydrogénation sur un dérivé aromatique nitré portant des atomes d'halogène liés au noyau aromatique, il se produit en même temps que la transformation du groupe nitro en groupe aminé un phénomène d'hydrogènolyse de la liaison carbone halogène pour donner d'une part le noyau déshalogèné et d'autre part des acides halohydriques.

Ce phénomène est connu depuis très longtemps puisqu'il a été décrit en 1904 par P. Sabatier et A. Mailhe.

De nombreux brevets ont été déposés pour éviter cette réaction secondaire tout en permettant de conserver une activité correcte au catalyseur.

Ces brevets peuvent être divisés en deux groupes : ceux utilisant comme catalyseur d'hydrogénation le nickel de Raney et ceux utilisant le platine ou le palladium.

Tous ces brevets décrivent l'utilisation d'un catalyseur modifié.

Dans le premier groupe de brevets décrivant l'utilisation du nickel de Raney on peut citer les brevets US 3067253, GB 1191610, J 73-49728, GB 1498722 et FR 2245615.

Le brevet US 3067253 décrit l'utilisation de nickel de Raney auquel est adjoint un hydroxyde de calcium ou de magnésium. Les températures réactionnelles sont néanmoins toujours basses (25 à 60°C) afin d'éviter la déshalogénation, ce qui ne permet pas de pouvoir utiliser ces procédés industriellement.

Le brevet GB 1191610 décrit l'utilisation du nickel de Raney associé à la présence d'un thiocyanate. Ce procédé ne permet qu'une hydrogénation lente (4 heures à 8 heures) et le catalyseur est altéré lors de l'hydrogénation, ce qui ne permet pas d'envisager un procédé en continu.

Le brevet J 73-49728 décrit l'utilisation du nickel de Raney associé à la présence d'une alkylamine, d'une alkanolamine ou d'une base hétérocyclique. Le brevet J 73-49728 décrit l'utilisation du nickel de Raney associé à la présence d'une alkylamine, d'une alkanolamine ou d'une base hétérocyclique. Dans ce brevet la température d'hydrogénation est limitée comme dans le brevet US précédemment mentionné à 60°C. Cette température ne permet pas une exploitation industrielle satisfaisante car la productivité du procédé à ces températures est insuffisante. Il est même indiqué dans le brevet FR 2245615 que le procédé du brevet J 72-49728 ne permet pas d'éviter la déhalogénation de façon satisfaisante puisque au moins 5 % de l'aniline obtenue est déshalogénée.

Le brevet GB 1498722 décrit l'utilisation de nickel de Raney avec un trialkylphosphite. La température de réaction est d'environ 100°C mais le taux de déshalogénation est très élevé puisqu'il varie entre 2 et 8 %. Ce procédé n'est donc pas envisageable industriellement.

Le dernier brevet décrivant l'utilisation du nickel de Raney est le brevet FR 2245615 qui l'associe avec un inhibiteur de deshalogénation choisi parmi le dicyandiamide, le cyanamide et le cyanamide calcique. La température de la réaction d'hydrogénation est comprise entre 50 et 130°C et les taux de déshalogénation sont toujours inférieurs à 0,15 %.

Dans le deuxième groupe de brevets décrivant l'utilisation de métaux de la mine du platine on peut citer les brevets FR 2330669 et FR 2127092.

Le brevet FR 2330669 décrit l'utilisation comme catalyseur d'hydrogénation de composés nitroaromatiques chlorés, du platine déposé sur charbon et inhibé par la présence d'un dérivé soufré choisi parmi les thioéthers et les disulfures. Le taux de déshalogénation est très faible (0,01 à 0,08 %).

Le brevet FR 2127092 décrit la préparation d'un catalyseur au platine déposé sur charbon qui est sulfuré. La préparation de ce catalyseur consiste à procéder d'abord à une hydrogénation du catalyseur puis à sulfurer ensuite celui-ci par addition d'hydrogène sulfuré selon une quantité variant entre 0,45 et 0,55 mole d'hydrogène sulfuré par mole d'hydrogène absorbée.

D'une part la préparation du catalyseur est difficile, d'autre part l'utilisation d'un catalyseur déposé sur charbon ne permet pas une décantation facile de la masse catalytique et rend ainsi très difficile l'exploitation du procédé selon un mode continu. L'utilisation de platine, catalyseur très onéreux a fait reculer l'industrie quant à la mise en oeuvre industrielle d'un tel procédé.

Outre les problèmes généraux ci dessus, il convient de mentionner les problèmes plus spécifiques des dérivés fluorés. Dans leur grande majorité, les anilines fluorées, qu'elles soient seulement fluorée ou qu'elles portent d'autre(s) halogène(s) sur le noyau aromatique, sont préparées par échange entre l'ion fluorure et un halogène de rang plus élevé ( c.à.d. dont le nombre atomique est plus élevé) à partir d'un dérivé nitré halogéné.

Quoique cette technique soit une des plus satisfaisante pour l'obtention de dérivés fluorés elle nécessite l'emploi de températures élevées et de solvants spécifiques le plus souvent des sulfones e/ou des sulfoxydes tels le sulfolane ou le diméthylsulfoxyde.

Un grand nombre de dérivés fluorés tels que les difluoro nitrobenzène sont difficile à séparer de ce type de solvant alors que l'aniline correspondante est aisée à séparer .

Comme les dérivés soufrés sont réputés empoisonner les catalyseurs d'hydrogénation le dérivé fluoré et nitré etait soigneusement purifié pour en éliminer toute trace de solvants avant l'hydrogénation. En outre les dérivés oxygénés du soufre sont relativement facilement hydrogènable pour donner d'une part de l'eau et d'autre part dérivés soufrés réduits tels que des thio ethers.

Les dérivés halogénés et plus particulièrement fluorés sont très sensible à l'hydrolyse pendant l'hydrogénation laquelle dégage deux molécules d'eau par fonction nitro.

C'est pourquoi un des buts de la présente invention est de fournir un nouveau procédé d'hydrogénation des dérivés aromatiques nitrés et halogénés notamment pour la synthèse des anilines halogénées

C'est pourquoi un autre but de la présente invention est de fournir un nouveau procédé d'hydrogénation des dérivé aromatiques nitrés et halogénés notamment pour la synthèse des anilines halogénées qui permette l'utilisation des mélanges réactionnels contenant lesdits dérivés chlorés et nitrés et provenant des réaction d'échange entre fluorure et halogène de rang plus élevé.

C'est pourquoi un autre but de la présente invention est de fournir un nouveau procédé d'hydrogénation des dérivé aromatiques nitrés et halogénés notamment pour la synthèse des anilines halogénées qui évite l'hydrogènolyse des liaisons carbone halogène.

La présente invention a su à partir du nickel ou équivalents, catalyseur d'hydrogénation le moins onéreux et aisément décantable, donc particulièrement bien adapté pour l'hydrogénation continue, l'associer avec un inhibiteur de déshalogénation de façon à lui apporter toutes les qualités requises pour l'hydrogénation des dérivés halogéno nitroaromatiques.

Elle consiste à mettre en présence le dérivé halogéno nitroaromatique ou polyhalogéno nitroaromatique avec un catalyseur d'hydrogénation comportant un métal de la colonne VIII appartenant à la première ligne des éléments de transition, avantageusement le nickel de préférence, le nickel de Raney et en présence d'un dérivé soufré.

Le dérivé soufré est choisi parmi les sulfures ou les hydrogéno sulfures, les sulfites et les hydrosulfites de métaux alcalins ou d'ammonium qui en milieu aqueux se dégradent en hydrogène sulfuré. Il peut aussi être choisi par les dérivés organiques du soufre.

On peut citer à titre d'exemples comme composés du soufre utilisable :
- l'hydrogène sulfureux
- le sulfure du sodium, de potassium, d'ammonium
- l'hydrosulfite de sodium ou dithionite de sodium ($Na_2S_2O_4$)
- le sulfite de sodium ($Na_2SO_3$)
- le tétrathionate de sodium ($Na_2S4O_6$)
- le tétrasulfure de sodium ($Na_2S4$)
- la thiourée
- les disulfures organiques
- les hétérocycliques soufrés tels que le thiophène le thioxane

On préfère utiliser la thiourée lorsque l'on ne désire pas utiliser des quantité élevée de composé du soufre .

Les dérivés halogéno nitroaromatiques qui peuvent être hydrogénés selon le procédé de la présente invention répondent à la formule suivante :

$(Z)_q(y)_p(x)_n$-Ar-NO$_2$

dans laquelle
- Ar représente un radical mono- ou poly-cyclique, homo- ou hétéro-cyclique, condensé ou non, éventuellement substitué par un groupe alkyle contenant 1 à 4 atomes de carbone.
- X,Y,Z représentent chacun un halogène choisi parmi le fluor, le chlore et le brome,
- n,p,q, représentent chacun un nombre entier compris entre 0 et 5.

La somme $n + p + q$ étant supérieure ou égale à 1 et inferieur ou égal à 7.

On préfère utiliser un composé halogéno nitroaromatique monocyclique contenant un à trois atomes d'halogène choisi parmi le chlore et le fluor fixés sur le noyau et tout particulièrement l'invention s'applique aux dérivés suivants :
- les chloronitrobenzènes
- les fluoronitrobenzènes
- les dichloronitrobenzènes

EP 0 409 709 A1

- les monochloro monofluoro nitrobenzènes
- les trichloronitrobenzènes
- les chloronitro alcoyl benzènes
- les fluoronitro alcoyl benzènes

On effectue l'hydrogénation des dérivés halogéno-nitroaromatiques à l'aide du nickel de Raney et de l'agent soufré dans les conditions usuelles d'hydrogénation. En raison du fort potentiel de non déshalogénation qu'apportent ces dérivés soufrés il est tout à fait possible de réaliser l'hydrogénation à une température comprise entre 50 et 150° C. Elle est réalisée préférentiellement entre 70 et 100° C ce qui permet d'obtenir des productivités (quantité d'amine formée par heure et par volume de milieu réactionnel) comparables avec celles d'amines non halogénées.

Un autre avantage du catalyseur selon l'invention que n'apportaient pas les catalyseurs déposés sur charbon est la facilité de sa mise en oeuvre par des procédés réalisés en continu. En effet la séparation du nickel de Raney est nettement plus facile que celle des catalyseurs déposés sur charbon.

Un dernier avantage du catalyseur selon l'invention est la facilité de sa mise en oeuvre, aucune préparation préalable du catalyseur n'est nécessaire comme dans le brevet FR 2127092 qui nécessite une hydrogénation du catalyseur suivi de sa sulfuration, dans le présent procédé il est seulement nécessaire d'introduire tous les réactifs dans le réacteur d'hydrogénation ; le nickel de Raney, le dérivé halogénonitroaromatique, le dérivé soufré, le solvant puis de pressuriser le réacteur avec de l'hydrogène.

Le procédé de l'invention peut être mis en oeuvre en l'absence de solvant ou dans tout solvant inerte dans les conditions de la réaction tel que par exemple :
- l'eau (en absence de risque d'hydrolyse important, cas des monofluorés),
- les alcools tels que le méthanol, l'éthanol, l'isopropanol
- les solvants aromatiques tels que le toluène, le xylène.
On préfère utiliser le méthanol.

Pour une meilleure mise en oeuvre de l'invention on préfère utiliser une quantité pondérale de nickel de Raney par litre de milieu réactionnel comprise entre 1 et 150 g de préférence entre 5 et 50 g et une quantité pondérale de dérivés soufrés calculée par rapport au nickel introduit comprise entre 0,2 et 10 %, la valeur supérieure notamment dans la cas des sulfones et des sulfoxydes étant essentiellement indicative. comme on le verra plus loin les sulfones et les sulfoxydes peuvent servir de solvant. Pour éviter l'hydrolyse les quantités de composés soufrés sont avantageusement plus importantes que celles nécessaires à l'inhibition de l'hydrogénolyse. Le rapport molaire entre les composés soufrés et le nickel est avantageusement au moins égal à environ 1 et de préférence 10.

Le procédé pouvant être mis en oeuvre en continu la quantité de dérivé halogéno nitroaromatique ne peut être établie de manière statique mais sous forme de flux. Ainsi des débits d'environ 1 à 3 moles par litre de milieu réactionnel et par heure sont tout à fait recommandés. La pression d'hydrogène est avantageusement comprise entre 1 et 100 bars de préférence 1 et 40 bars et encore plus préférentiellement entre 5 et 25 bars.

Lorsque l'on utilise la présente invention pour traiter les mélanges réactionnels issus de l'échange entre fluor et halogène de rang plus élevé, il suffit d'ajouter un catalyseur à base de nickel au mélange réactionnel après éventuelle filtration pour éliminer les sels d'acides halohydriques et de mener les réactions d'hydrogénation dans les conditions ci-dessus. le solvant sera alors le même que celui utilisé pendant la réaction d'échange à savoir les solvants soufrés oxygénés. On peut toutefois prévoir une dilution avec les solvants utilisables lors de l'hydrogénation tels que ceux cités précédemment.

Un autre but de la présente invention est de fournir un nouveau réactif d'hydrogénation des dérivé aromatiques nitrés et halogénés notamment pour la synthèse des anilines halogénées qui évite l'hydrolyse et/ou l'hydrogènolyse des liaisons carbone halogène.

ce but est atteint par un réactif d'hydrogénation comportant :
- un catalyseur d'hydrogénation à base de nickel ou un métal de la colonne VIII appartenant à la première ligne des éléments de transition, avantageusement le nickel de préférence, le nickel de Raney,
- un solvant organique soufré oxygéné ;
et
- de l'hydrogène .
Par solvant organique soufré oxygéné il faut comprendre tout solvant organique
- inerte vis à vis des des autre constituants du réactif et vis à vis des substrats ;
- liquide à la température où a lieu la réaction , avantageusement dont le point de fusion commençante est inférieur à 100° C et de préférence dont le point d'ébullition finissante est supérieur à 100° C ;
- présentant (au moins) une fonction $-SO_2-$ ou $-SO-$.dans au moins un de ses constituants principaux lorsque le solvant est un mélange.

4

Comme paradigmes des solvants organiques soufrés oxygénés on peut citer le diméthylsulfoxyde (D.M.S.O.) et le sulfolane.

Comme cela apparaît de résultats ci-après il a été montré que l'utilisation comme solvant, ou comme élément constitutif du solvant, des sulfones et des sulfoxydes, réduisait le taux d'hydrolyse et d'hydrogènolyse, permettait l'emploi de mélange réactionnel brut provenant de réaction d'échange halogène fluor lorsque le solvant est constitué de sulfone(s) et/ou de sulfoxyde(s) seul(s) ou en mélange avec des solvants ne pertubant pas la réaction d'hydrogénation

La présente invention sera plus complètement décrite à l'aide des exemples suivants qui ne doivent en aucun cas être considérés comme limitatifs de l'invention.

Exemple 1

Dans un autoclave de 750 ml en acier inox, on charge 6 g de nickel de Raney, 0,3 g de thiourée et 300 ml de méthanol à 95 %. Après fermeture, le réacteur est purgé plusieurs fois avec de l'azote puis de l'hydrogène.

La pression est fixée à 20 bars. Le mélange réactionnel est chauffé à la température de 100°C. Dès que celle-ci est atteinte, on procède à l'injection du substrat (mélange para-fluoronitrobenzène/ortho-fluoronitrobenzène de composition molaire 87%/13 %) pendant 22 minutes soit 40 ml (377 mmoles).

En fin de réaction, le réacteur est refroidi à température ambiante puis dégazé.

La masse réactionnelle est filtrée sur fritté et le filtrat est analysé en CPG.

La distribution des produits est la suivante :

TT = 100 %
para-fluoro aniline : 330,4 mmoles = => RR = 87,6 %
ortho-fluoro aniline : 47,7 mmoles = => RR = 12,7 %
aniline < 0,05%

L'activité du catalyseur est de 0,171 mole substrat/g Nickel x h.

Exemple comparatif 1

On procède de la même façon à l'hydrogénation du mélange para-ortho fluoronitrobenzène mais sans ajout de thiourée (quantités identiques).

Le dosage en fin de réaction donne :

TT = 100 %
para-fluoro aniline : 270 mmoles = => RR = 71,6 %
ortho-fluoro aniline : 27,6 mmoles = => RR = 7,3 %
aniline : 74,6 mmoles = => RR = 19,8 %
écart au bilan = - 1,3 %

Exemple 2 : Hydrogénation du 3,4-dichloronitrobenzène

Pied - : MeOH 90 % = 100 ml
Ni Raney = 2 g
(ajout) : (voir tableau)
Substrat : dichloronitrobenzène : 25 g (130 mmoles)
dissous dans 125 cc MeOH pur (volume = 138 cc)

Dans un autoclave de 300 ml HASTELLOY, on charge le pied contenant le solvant, le catalyseur et éventuellement un ajout ou un agent neutralisant tel qu'un tampon composé d'acétate de soude et d'acide acétique. Après fermeture le réacteur est purgé plusieurs fois à l'azote, puis à l'hydrogène, la pression est fixée à 16,5 bars (celle-ci atteint 18 bars à 75°C).

Ce pied est agité et chauffé à la température de 75°C. Dès que celle-ci est atteinte, on procède à l'injection du substrat pendant 30 mn. En fin de réaction le réacteur est refroidi à température ambiante puis dégazé.

La masse réactionnelle est filtrée sur fritté et le filtrat est analysé en chromatographie phase gazeuse (CPG).

5

## RESULTATS

| Ni/RANEY | AJOUT | TT | RR % 3-4 DCA | Aniline | 3-ClA 4-ClA | Cl - % Molaire |
|---|---|---|---|---|---|---|
| 2g (20g/L) | ACOH : 0,8 g NaOH : 1,6 g | 100 | 90 | 6 | 3,5 | 15,6 |
| 2g (20g/L) | Thiourée : 0,1 g (1 g/L) | 100 | 99,3 | – | 0,3 | 0,4 |
| 2g (20g/L) | Na$_2$S,9H$_2$0 0,325 g 3,25 g/L | 100 | 97,3 | 0,9 | 0,9 | 2,7 |

## Exemple 3 : Hydrogénation du 3-Cl, 4-F Nitrobenzène

On procède de la même manière qu'à l'exemple 1 en introduisant initialement
( MeOH (90 %) = 300 ml
( Nickel de Raney = 6 g
( Thiourée = 0,9 g
puis par injection 3-Cl, 4-F Nitrobenzène = 21 g (0,120 mole)
On établit une pression d'hydrogène de 20 bars et on chauffe le milieu réactionnel à 100° C.
Après analyse par chromatographie en phase gazeuse on montre que le taux de transformation du 3-Cl, 4-F nitrobenzène est complet et que l'hydrodéchloration est de 0,7 %.

## Exemple 4 : Synthèse du difluoro - 2,4 nitrobenzène

Dans un réacteur de 1l, préchauffé à 50° C et placé sous atmosphère d'azote, est chargé :
- 174 g de fluorure de potassium (teneur en eau inférieur à 100 ppm)
- 13 g de chlorure de tretraméthylammonium
et 176 g de diméthylsulfoxide (contient 50 ppm d'eau)
puis on introduit 230,4 g de dichloro-2,4 nitrobenzène.
Le milieu réactionnel est agité, de manière à ce que tout le solide soit en suspension, pendant 6h à 130° C. Le réacteur est alors refroidi à 30° C, le milieu réactionnel est dilué à l'aide de 100 ml de chlorure de méthylène. Le mélange agité dix minutes et transvasé sur un verre filtré. Le réacteur et le précipité sont rincés deux fois par 100 ml de chlorure de méthylène. Le filtrat est récupéré et le chlorure de méthylène est évaporé à l'aide d'un évaporateur rotatif ( bain = 70° C,p = 100 mmHg). On obtient ainsi 375,2 g de produit : conversion 100 %, rendement en difluoro-2,4 nitrobenzène 91 % (pureté 99 %). Celui est divisé en trois parties. La première (fraction A) sera utilisée telle quelle. Les deux autres parties sont lavées à l'eau pour éliminer le DMSO.
Celui-ci est récupéré à 98 % dans la phase aqueuse. La seconde partie (fraction B) sera utilisée sans autre purification alors que la troisième partie (fraction C) est purifiée par distillation.

## Exemple 5 : Synthèse du difluoro-2,4 nitrobenzène.

Le protocole décrit dans l'exemple 4 est repris en utilisant dans un réacteur de 1l :
- 208,8 g de fluorure de potassium (teneur en eau inférieur à 100ppm)

- 6 g de fluorure de césium
- 276,5 g de dichloro-2,4 nitrobenzène

et 215 g de sulfolane.

Après 11 h de chauffage à 180° C, on obtient conversion = 97,3 %,

rendement en difluoro-2,4 nitrobenzène = 66,5 %

rendement en fluoro-2-chloro-4 nitrobenzène = 4,6 %

rendement en fluoro-4-chloro2-nitrobenzène = 26,2 %.

Le brut réactionnel est divisé en deux parties. La première (fraction D) sera utilisée telle quelle. La seconde est rectifiée et conduit à un mélange de difluoro-2,4 nitrobenzène 69 %, fluoro-2-chloro-4 nitrobenzène = 4,6 %, et fluoro-4-chloro-2-nitrobenzène 26,4 % (fraction E).

## Exemple 6

Dans un tube en verre de 30 ml, on chage 0,18 g (3 mmoles) de Nickel de Raney, 15,2 g (15 mmoles de difluoro-2,4- nitrobenzène) de substrat (fraction A) et 15 ml d'éthanol absolue. Le tube est placé dans un autoclave cylindrique de 250 ml qui est placé sous atmosphère d'hydrogène. L'autoclave est agité 1h10 à 60° C sous 20 bars d'hydrogène. La solution aqueuse obtenue après un traitement traditionnel est dosé par chromatographie phase vapeur :

- conversion 44,7 %
- sélectivité en difluoro-2,4 aniline : 74,2 %

## Exemple 7 à 12 :

Le protocole de l'exemple 3 est repris avec les différentes fractions (cf exemples 1 et 2) dans les condition suivantes

| N° d'exemple | Onium Substrat | Conditions réactionnelles nelles | Durée | Composé additionnel | Conversion du DiF24NB (%) | Conversion du DiF24NA (%) |
|---|---|---|---|---|---|---|
| 7 | Fraction A | pH$_2$ = 20 bars-60° C | 3 h | - | 69,6 | 89,8 |
| 8 | Fraction B | pH$_2$ = 20 bars-60° C | 1h10 | - | 75,7 | 79,1 |
| 9 | Fraction C | pH$_2$ = 20 bars-60° C | 1h10 | - | 74,4 | 61,5 |
| 10 | Fraction C | pH$_2$ = 20 bars-60° C | 1h10 | DMSO - 5% | 91,2 | 81,4 |
| 11 | Fraction D | pH$_2$ = 20 bars-60° C | 1h10 | - | 47,2 | 75,9 |
| 12 | Fraction E | pH$_2$ = 20 bars-60° C | 1h10 | - | 69,7 | 53,4 |

## Exemple 13

La réaction d'hydrogénation est réalisée par addition en continu de 80 mmoles de difluoro-2,4 nitrobenzène (fraction A), en solution dans 75 ml d'éthanol sur un peid contenant 1g de Nickel de Raney (16 mmole) dans 75 ml d'éthanol, à 60° C sous une pression d'hydrogène de 20 bars.

L'addition dure 4h40. On obtient les résultats suivants :

- conversion du difluoro-2,4 nitrobenzène 100 %
- sélectivité en dilfuoro-2,4 aniline 90 %

## ROLE DES SULFONES ET DES SULFOXYDES DANS LA PREVENTION DE L'HYDROLYSE

L'analyse des mélanges réactionnels après l'hydrogénation le rôle t très favorable de ces composés utilisés comme solvant, notamment des sulfoxydes, les principaux résultats sont réunis dans le tableau suivant.

| N° d'exemple | DMSO Ni Raney (% molaire | TT (%) | RT (%) DiF$_2$4A | RT (%) F(OH)A |
|---|---|---|---|---|
| 4 | 10 | 69,6 | 89,8 | 2,8 |
| 5 | 0 | 75,7 | 79,1 | 16,7 |
| 6 | 0 | 74,4 | 61,5 | 15,9 |
| 7 | 1 Sulfolane/Ni | 91,2 | 81,4 | 13,5 |
| 8 | 10 Sulfolane/Ni | 47,9 | 75,9 | 7,9 |
| 9 | 0 DMSO/Ni | 69,7 | 53,4 | 10,6 |
| 10 | 10 | 100 | 90 | 2 |

**Revendications**

1. Procédé de préparation des dérivés halogénoaminoaromatiques caractérisé en nitroaromatique avec du nickel de Raney et de l'hydrogène en présence d'un dérivé soufré.

2. Procédé selon la revendication 1, caractérisé en ce que le dérivé halogénonitroaromatique répond à la formule (I)

(Z)$_q$ (Y)$_p$ (X)$_n$- Ar -NO$_2$

dans laquelle

- Ar représente un dérivé aromatique mono, poly ou hétérocyclique éventuellement substitué par un groupe alkyle contenant 1 à 4 atomes de carbone

- X, Y et Z représentent un halogène choisi parmi le fluor, le chlore et le brome

- n, p et q représentent un nombre entier compris entre 0 et 5 la somme n + p + q pouvant être égale ou supérieure à 1.

3. Procédé selon la revendication 2, caractérisé en ce que Ar représente un radical aromatique monocyclique et X et Y représentent le chlore et/ou le fluor et la somme n + p est égale ou supérieure à 1 et inférieure ou égale à 3.

4. Procédé selon l'une des revendications 1 à 6, caractérisé en ce que le dérivé soufré est choisi parmi les sulfures, les hydrogénosulfures, les sulfites, les hydrogénosulfites de métaux alcalins ou d'ammonium ainsi que parmi les dérivés organiques du soufre.

5. Procédé selon la revendication 4, caractérisé en ce que les dérivés organiques du soufre sont choisis parmi les disulfures, le thiophène, le thioxane et la thiourée.

6. Procédé selon la revendication 5, caractérisé en ce que le dérivé organique du soufre est la thiourée.

7. Procédé selon l'une des revendications 1 à 6, caractérisé en ce que la réaction est réalisée en l'absence de solvant ou dans un solvant choisi parmi les alcools, les dérivés aromatiques.

8. Procédé selon la revendication 7, caractérisé en ce que le solvant est le méthanol.

9. Procédé selon l'une des revendications 1 à 8, caractérisé en ce que la quantité de nickel utilisée est comprise entre 1 et 150 g par litre de milieu réactionnel et de préférence entre 5 et 50 g.

10. Procédé selon l'une des revendications 1 à 9, caractérisé en ce que la quantité pondérale de dérivés soufrés calculée par rapport au nickel est comprise entre 0,2 et 10 %.

11. Procédé selon l'une des revendications 1 à 10, caractérisé en ce que la température de la réaction est comprise entre 50° C et 150° C et de préférence entre 70° C et 100° C.

12. Procédé selon l'une des revendications 1 à 4 et 7 à 11, caractérisé en ce que le dérivé soufré est choisi parmi les sulfones et les sulfoxydes.

13. Procédé selon la revendication 12, caractérisé en ce que la réaction est réalisée en l'absence de solvant ou avec le dérivé soufré comme solvant.

14. Procédé selon l'une des revendications 12 et 13, caractérisé en ce que la quantité de nickel utilisée est comprise entre 1 et 150 g par litre de milieu réactionnel et de préférence entre 5 et 50 g.

15. Procédé selon l'une des revendications 12 à 14, caractérisé en ce que la température de la réaction est comprise entre 50°C et 150°C et de préférence entre 70°C et 100°C.

16. Réactif d'hydrogénation comportant :
- un catalyseur d'hydrogénation à base de nickel avantageusement du nickel de Raney ;
- un solvant organique soufré oxygéné ;
et
- de l'hydrogène avantageusement à une pression au moins égale à 5 bars.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 002 802 (BASF)<br>* page 5; page 6, lignes 1-11; page 6, lignes 25-35; page 7, lignes 9-19; page 11; exemple 4; revendications *<br>--- | 1-3,7,8,10,11,14,15 | C 07 C 209/36<br>C 07 C 211/52 |
| X | EP-A-0 000 805 (THE CLAYTON ANILINE CIE.)<br>* résumé; page 3, lignes 6-22; page 4, lignes 5-12; revendications 1,2,4-6,9,10 *<br>--- | 1-5,7-11,14,15 | |
| X | DE-A-1 643 379 (FARBENFABRIKEN BAYER AG)<br>* pages 2-5; revendication *;<br>GB-A-1191610 (cat. D)<br>--- | 1-3,7-11 | |
| A,D | FR-A-2 330 669 (BAYER)<br>* page 6; revendications 1-4 *<br>--- | 1-5 | |
| A | DE-A-2 105 780 (FARBWERKE HOECHST AG)<br>* page 1, lignes 7-37; page 3, exemples 1-5; revendications 1,7-9 *<br>--- | 1-5 | |
| A | DE-A-2 105 682 (FARBWERKE HOECHST AG)<br>* page 5; revendications 1,2 *<br>--- | 1-5,11 | DOMAINES TECHNIQUES RECHERCHES (Int. Cl.5)<br><br>C 07 C 209/00<br>C 07 C 211/00 |
| A | US-A-3 989 756 (M. FUJISE)<br>* revendications 1,5,6,8 *; &<br>FR-A-2245615 (cat. D)<br>----- | 1,2,11 | |

Le présent rapport a été établi pour toutes les revendications

| Lieu de la recherche | Date d'achèvement de la recherche | Examinateur |
|---|---|---|
| BERLIN | 22-08-1990 | RUFET J.M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P0402)